# EUROPEAN PATENT APPLICATION

(11) **EP 3 029 464 A1**
(43) Date of publication of application: **08.06.2016**
(21) Application number: 14832954.3
(22) Date of filing: 22.07.2014
(51) Int. Cl.: G01N 33/564, C07K 14/47, C12Q 1/02, G01N 33/15, G01N 33/50, G01N 33/53, C12N 15/09

(54) **METHOD FOR ASSESSING AUTOIMMUNE ARTHRITIS, AND SCREENING METHOD FOR SUBSTANCES INHIBITING ACTIVITY OF AUTOIMMUNE ARTHRITIS-INDUCING T CELLS**

(30) Priority: 31.07.2013 JP 2013159815
(71) Applicant: Kyoto University, Kyoto-shi, Kyoto 606-8501 (JP)
(72) Inventor: SAKAGUCHI, Shimon, Kyoto-shi Kyoto 606-8501 (JP); ITO, Yoshinaga, Kyoto-shi Kyoto 606-8501 (JP)
(74) Representative: Witte, Weller & Partner Patentanwälte mbB
(86) International application number: PCT/JP2014/069306
(87) International publication number: WO 2015/016096

(57) **Abstract**

Provided are a method for identifying the presence or likelihood of autoimmune arthritis, the method comprising detecting (1) an anti-ribosomal protein L23a antibody or (2) ribosomal protein L23a-reactive CD4⁺ T cells in a sample derived from a subject; and a method for screening for a substance that inhibits the activation of autoimmune arthritogenic T cells, the method comprising the steps of bringing a test substance into contact with ribosomal protein L23a-reactive T cells, bringing the T cells into contact with ribosomal protein L23a or a fragment thereof, measuring the level of a proinflammatory cytokine secreted from the T cells, and analyzing the test substance-dependent change in the secretion level of the proinflammatory cytokine.

## Description

### TECHNICAL FIELD

The present invention relates to a method for identifying autoimmune arthritis and a method for screening for an inhibitor of activation of autoimmune arthritogenic T cells.

### BACKGROUND ART

Rheumatoid arthritis is an autoimmune disease that causes chronic inflammation and tissue destruction mainly on the joints of the whole body. Rheumatoid arthritis affects about 1% of the total population. The clinical features of rheumatoid arthritis are classified into two categories: a pathological autoimmune reaction by acquired-immune cells (T cells and B cells), and chronic inflammation by proinflammatory cytokines. Anti-cytokine therapy on proinflammatory cytokines has recently been successful in terms of adequately controlling the cytokines. However, the therapy is insufficient to rectify the pathological autoimmune reaction, which raises significant problems of recrudescence of the disease after anti-cytokine therapy is stopped. CD4⁺ T cells have been reported to serve a pivotal role in the pathological autoimmune reaction observed in rheumatoid arthritis, but self antigens recognized by the CD4⁺ T cells that induce the disease have long been unknown, which has hindered antigen-specific control of the autoimmune reaction as well as the analysis of the disease-inducing CD4⁺ T cells.

To date, self antigens recognized by the T cells that induce autoimmune arthritis have been found only in the autoimmune arthritis system induced by special immunization and in the autoimmune arthritis system that indispensably requires B cells and autoantibody. For example, arthritis has been reported to be induced in DBA/1 mice by subcutaneous administration of 100 mg of type II collagen with CFA (complete Freund's adjuvant) followed by subcutaneous administration 20 days later of 100 mg of type II collagen with IFA (incomplete Freund's adjuvant) (type II collagen-induced arthritis, see Non Patent Literature 1). This system has been found to require CD4⁺ T cells. However, arthritis has also been reported to be induced by anti-type II collagen autoantibody produced in the body as a result of immunization. Consequently, it has been unclear whether type II collagen-specific CD4⁺ T cells induce arthritis in the absence of autoantibody.

In K/BxN mice spontaneously developing an autoimmune disease with aging, glucose phosphate isomerase (GPI) has been defined as a targeted self antigen (see Non Patent Literature 2 and 3). In this system, the activation of B cells by self-reactive CD4⁺ T cells results in the production of autoantibody against GPI, and the autoantibody induces arthritis. However, the self-reactive CD4⁺ T cells in this system do not induce arthritis in the absence of the autoantibody.

There still are many unknown factors contributing to autoimmune arthritis including rheumatoid arthritis. Finding a new self antigen that induces autoimmune arthritis is an important task. In particular, a self antigen in autoimmune arthritis induced in a CD4⁺ T cell-dependent and autoantibody-independent manner has not been reported yet, and finding such a self antigen is expected to enable the mechanistic analysis of autoimmune arthritis and the development of therapy.

### CITATION LIST

### NON PATENT LITERATURE

Non Patent Literature 1: Courtenay JS, Dallman MJ, Dayan AD, Martin A, Mosedale B. Immunisation against heterologous type II collagen induces arthritis in mice. Nature 1980; 283:666-8
Non Patent Literature 2: Kouskoff, V. et al. Organ-specific disease provoked by systemic autoimmunity. Cell 87, 811-822 (1996).
Non Patent Literature 3: Matsumoto, I., Staub, A., Benoist, C. & Mathis, D. Arthritis provoked by linked T and B cell recognition of a glycolytic enzyme. Science 286, 1732-1735 (1999).

### SUMMARY OF INVENTION

### TECHNICAL PROBLEM

An object of the present invention is to find a self antigen in autoimmune arthritis induced in a CD4⁺ T cell-dependent and autoantibody-independent manner. Another object is to provide a method for identifying the presence or likelihood of autoimmune arthritis by using the self antigen, and a method for screening for a substance that inhibits the activation of CD4⁺ T cells that react with the self antigen and induce T cell-dependent autoimmune arthritis.

### SOLUTION TO PROBLEM

The present invention was made to solve the above problems and includes the following.
[1] A method for identifying the presence or likelihood of autoimmune arthritis, the method comprising detecting (1) an anti-ribosomal protein L23a antibody or (2) ribosomal protein L23a-reactive CD4⁺ T cells in a sample derived from a subject.
[2] The identification method according to the above [1], wherein the ribosomal protein L23a is a protein consisting of an amino acid sequence of SEQ ID NO: 1.
[3] The identification method according to the above [1] or [2], wherein the subject is anti-CCP antibody negative.
[4] Use of ribosomal protein L23a or a fragment thereof for the identification of the presence or likelihood of autoimmune arthritis, the use comprising detecting an anti-ribosomal protein L23a antibody or ribosomal protein L23a-reactive CD4⁺ T cells by using ribosomal protein L23a or a fragment thereof.
[5] The use according to the above [4], wherein the ribosomal protein L23a is a protein consisting of an amino acid sequence of SEQ ID NO: 1.
[6] The use according to the above [4], wherein the ribosomal protein L23a fragment comprises the amino acids at positions 71 to 90 of SEQ ID NO: 1.
[7] A method for screening for a substance that inhibits the activation of autoimmune arthritogenic T cells, the method comprising the steps of
   bringing a test substance into contact with ribosomal protein L23a-reactive T cells,
   bringing the T cells into contact with ribosomal protein L23a or a fragment thereof, measuring the activation level of the T cells, and
   analyzing the test substance-dependent change in the activation level of the T cells.
[8] The screening method according to the above [7], wherein the step of measuring the activation level of the T cells comprises measuring the level of a proinflammatory cytokine secreted from the T cells, the level of proliferation of the T cells or the level of an activated surface marker of the T cells.
[9] The screening method according to the above [7] or [8], wherein the ribosomal protein L23a is a protein consisting of an amino acid sequence of SEQ ID NO: 1.
[10] The screening method according to the above [7] or [8], wherein the ribosomal protein L23a fragment comprises the amino acids at positions 71 to 90 of SEQ ID NO: 1.
[11] A ribosomal protein L23a fragment consisting of part of an amino acid sequence of SEQ ID NO: 1 and comprising at least 5 contiguous amino acid residues of the amino acid sequence of SEQ ID NO: 1.
[12] The fragment according to the above [11], comprising the amino acids at positions 71 to 90 of SEQ ID NO: 1.

### ADVANTAGEOUS EFFECTS OF INVENTION

The present invention provides a method for identifying the presence or likelihood of autoimmune arthritis. The present invention also provides a method for screening for a substance that inhibits the activation of CD4⁺ T cells that induce T cell-dependent autoimmune arthritis. The present invention further provides a protein fragment suitable for use in these methods and in study of autoimmune arthritis.

### BRIEF DESCRIPTION OF DRAWINGS

Fig. 1 is the photographs of a retrogenic mouse that has developed arthritis.
Fig. 2 is a table showing the amino acid sequences of an arthritogenic T cell receptor.
Fig. 3 is a chart showing the activation of the arthritogenic T cell receptor by ribosomal protein L23a.
Fig. 4 is a chart showing the identification of an antigen fragment of ribosomal protein L23a.
Fig. 5 is a photograph showing the detection of an anti-ribosomal protein L23a antibody in the serum of retrogenic mice expressing the arthritogenic T cell receptor.
Fig. 6 is a chart showing the detection of an anti-ribosomal protein L23a antibody in the sera of rheumatoid arthritis patients.
Fig. 7 is a chart showing the detection of ribosomal protein L23a-reactive T cells in the synovial fluid of a rheumatoid arthritis patient.
Fig. 8 is a chart showing the detection of an anti-ribosomal protein L23a antibody in the sera of anti-CCP antibody-negative rheumatoid arthritis patients.

### DESCRIPTION OF EMBODIMENTS

The inventors have established SKG mice (JP Patent No. 3467520), which develop autoimmune arthritis very similar to rheumatoid arthritis, and have conducted further research. SKG mice have a point mutation of ZAP-70, a signaling molecule in T cells. This mutation alters thymic selection of T cells to allow the presence of peripheral self-reactive T cells that are causative of arthritis, and results in the development of arthritis (Sakaguchi, N. et al. Altered thymic T-cell selection due to a mutation of the ZAP-70 gene causes autoimmune arthritis in mice. Nature 426, 454-460, doi:10.1038/nature02119 (2003), Hirota, K. et al. T cell self-reactivity forms a cytokine milieu for spontaneous development of IL-17+ Th cells that cause autoimmune arthritis. The Journal of experimental medicine 204, 41-47, doi:10.1084/jem.20062259 (2007)). SKG mouse arthritis is CD4⁺ T cell dependent. That is, introduction of CD4⁺ T cells into immunodeficient mice results in the development of arthritis in the recipient mice.

The inventors, as shown in Examples described later, used SKG mice to identify CD4⁺ T cells that induce arthritis independently of autoantibodies. The inventors then attempted to determine the antigen recognized by the CD4⁺ T cells. The inventors, as a result, identified ribosomal protein L23a as the self antigen that induces CD4⁺ T cell-dependent autoimmune arthritis. The inventors conducted further studies on the self antigen and completed the present invention.

### Method for identifying autoimmune arthritis

The present invention provides a method for identifying the presence or likelihood of autoimmune arthritis. The identification method of the present invention is conducted by detecting (1) an anti-ribosomal protein L23a antibody or (2) ribosomal protein L23a-reactive CD4⁺ T cells in a sample derived from a subject.

The autoimmune arthritis to be identified by the method of the present invention may be any type of arthritis as long as it is induced by an autoantibody or self-reactive T cells. Examples of such autoimmune arthritis include rheumatoid arthritis, reactive arthritis, psoriatic arthritis, ankylosing spondylitis, collagen disease-associated arthritis, etc. The autoimmune arthritis to be identified is preferably rheumatoid arthritis.

The subject is not particularly limited, but preferred are those who have developed arthritis or those who are suspected of developing arthritis. The subjects who are suspected of developing arthritis are, for example, those who manifest subjective symptoms of arthritis. Preferred are subjects who are anti-CCP antibody negative. In particular, preferred are subjects who have developed arthritis but are anti-CCP antibody negative, or subjects who are suspected of developing arthritis but are anti-CCP antibody negative.

The sample derived from a subject is not particularly limited as long as the sample contains an antibody or T cells, but preferred are body fluids, such as blood, serum, plasma, tissue fluid, lymph fluid, cerebrospinal fluid, pus, mucus, nasal mucus, sputum, urine, feces and ascites fluid.

The antibody against ribosomal protein L23a (hereinafter called "L23a") may be any antibody that specifically binds to L23a. The L23a-reactive CD4⁺ T cells may be any CD4⁺ T cells that are activated by specifically reacting with L23a.

The detection of the anti-L23a antibody and the L23a-reactive CD4⁺ T cells can be achieved using L23a or a fragment thereof. The source of L23a to be used is not particularly limited, but preferred is mammalian L23a. Preferred mammals are humans, chimpanzees, monkeys, dogs, cattle, sheep, rabbits, mice, rats, guinea pigs, etc., and more preferred are humans. L23a is well conserved among mammals, and the same L23a amino acid sequence (SEQ ID NO: 1) is shared by, at least, humans, rhesus monkeys, cattle, sheep, rats and mice. L23a amino acid sequences of various animals and the nucleotide sequences of the genes encoding them are available from, for example, known databases (DDBJ, GenBank, EMBL, etc.) under the accession numbers shown in Table 1 below.

**Table 1**

| | Amino acid sequence | Nucleotide sequence |
|---|---|---|
| Human | NP_000975 | NM_000984 |
| Rhesus monkey | NP_001180494 | NM_001193565 |
| Cattle | NP_001039423 | NM_001045958 |
| Sheep | NP_001171148 | NM_001177677 |
| Rat | NP_001101753 | NM_001108283 |
| Mouse | NP_997406 | NM_207523 |
| Rabbit | NP_001192153 | NM_001205224 |

The L23a fragment is not particularly limited as long as it consists of part of L23a, but preferably the fragment consists of part of mammalian L23a, more preferably the fragment is part of L23a consisting of the amino acid sequence of SEQ ID NO: 1. The total number of the amino acid residues of the L23a fragment is not particularly limited but is preferably 5 or more. The maximum number of the amino acid residues is not particularly limited, but preferably the total number of the amino acid residues is about 140 or less, more preferably about 120 or less, further preferably about 100 or less, further preferably about 80 or less, further preferably about 60 or less, further preferably about 40 or less, particularly preferably about 20 or less.

The L23a fragment is preferably a fragment comprising the amino acids at positions 71 to 90 (LDHYAIIKFPLTTESAMKKI; SEQ ID NO: 3) of the amino acid sequence of L23a (SEQ ID NO: 1), and more preferably is a fragment consisting of the amino acid sequence of SEQ ID NO: 3.

L23a and a fragment thereof can be produced, for example, through known gene engineering techniques, by constructing a recombinant expression vector into which a gene encoding L23a or a fragment thereof has been inserted in such a manner that the gene is expressed, introducing the vector into an appropriate host cell to allow the gene to be expressed as a recombinant protein, and purifying the protein. Alternatively, L23a and a fragment thereof can be produced, for example, using an in vitro transcription and translation system. Further alternatively, L23a and a fragment thereof can be produced by solid-phase synthesis (Fmoc or Boc method) or liquid-phase synthesis in accordance with known conventional peptide synthetic protocols.

The method for detecting an anti-L23a antibody is not particularly limited, and the antibody can be detected by an appropriately selected known method for detecting an antibody that specifically binds to an antigen. A suitable detection method is, for example, ELISA. In cases where ELISA is used to detect an anti-L23a antibody, L23a or a fragment thereof is immobilized on a plate, then the serum from a subject is added to the plate to allow the reaction to proceed, and an appropriate secondary antibody is added to detect an anti-L23a antibody.

The criteria for determining a subject as being anti-L23a antibody-positive can be defined, for example, based on the amount of an anti-L23a antibody in a healthy individual without arthritis (for example, a human that does not manifest subjective symptoms of arthritis) as a control. For example, the amount of an anti-L23a antibody is measured in controls in the same manner as in the detection of the antibody in a subject; then the reference value, defined as the mean + 3SD of the controls, is calculated from the mean value and the standard deviation (SD) of the controls; and a subject of which the amount of an anti-L23a antibody is less than or equal to the reference value is determined as normal, and a subject of which the amount of an anti-L23a antibody is more than the reference value is determined as positive. However, the determination process is not limited to the above. Alternatively, the amounts of the antibody in controls may be collected as background data, and the reference value may be calculated from the accumulation of the data.

The method for detecting L23a-reactive CD4⁺ T cells is not particularly limited, and the T cells can be detected by an appropriately selected known method for detecting T cells that specifically react with an antigen. Suitable methods therefor include, for example, the detection of cells that react with L23a and secrete a proinflammatory cytokine, the detection of the proliferation of L23a-reactive CD4⁺ T cells, and the detection of an activated surface marker (CD154 etc.). Specifically, for example, CD4⁺ T cells are separated from the blood of a subject by a known method, then the cells are brought into contact with L23a or a fragment thereof, and the cells that secrete a proinflammatory cytokine are detected with a flow cytometer. Examples of the proinflammatory cytokine include IFN-γ, TNF-α, IL-1, IL-6, IL-8, IL-12, IL-17, IL-18, etc.

The detection of L23a-reactive CD4⁺ T cells in a subject confirms that the subject is positive for L23a-reactive CD4⁺ T cells.

The positive results for at least either of an anti-L23a antibody and L23a-reactive CD4⁺ T cells in a subject confirm that the subject has developed autoimmune arthritis or is likely to develop autoimmune arthritis. As described, the method of the present invention identifies the presence or likelihood of autoimmune arthritis, which allows early and effective therapeutic intervention or identification of the presence of the disease through more careful follow-up check-ups.

Further, the Examples described later showed that an anti-L23a antibody was detected in the sera of more than half of the anti-CCP antibody-negative rheumatoid arthritis patients. Hence, the identification method of the present invention can be applied to subjects who have developed arthritis but are anti-CCP antibody negative, or subjects who are suspected of developing arthritis but are anti-CCP antibody negative. The identification method of the present invention is therefore very useful for identifying the presence or likelihood of autoimmune arthritis in such subjects.

### Screening method

The present invention also provides a method for screening for a substance that inhibits the activation of autoimmune arthritogenic T cells. The screening method of the present invention comprises the steps of bringing a test substance into contact with L23a-reactive T cells, bringing the T cells into contact with L23a or a fragment thereof, measuring the activation level of the T cells, and analyzing the test substance-dependent change in the activation level of the T cells. The substance selected by the screening method of the present invention is useful as an active ingredient of a prophylactic or therapeutic agent for autoimmune arthritis.

The test substance to be subjected to the screening method of the present invention is, for example, a nucleic acid, a peptide, a protein, a non-peptidic compound, a synthetic compound, a fermentation product, a cell extract, a cell culture supernatant, a plant extract, a mammalian tissue extract, a plasma, or the like. The test substance may be a novel substance or a known substance.

The T cells reactive with L23a (hereinafter called "L23a-reactive T cells") can be collected from an autoimmune arthritis patient in the same manner as in the method for detecting L23a-reactive CD4⁺ T cells. Alternatively, the L23a-reactive T cells can be produced by introducing the gene of the T cell receptor that specifically recognizes L23a into a T cell line deficient in the expression of T cell receptors (for example, 5KC-73.8.20) (see Examples).

In the step of bringing a test substance into contact with L23a-reactive T cells, the test substance may be brought into contact with the L23a-reactive T cells by any method and the method is not particularly limited as long as the test substance is allowed to be in contact with the T cells. The test substance may be brought into contact with the T cells by, for example, adding the test substance to the medium in which the L23a-reactive T cells are cultured, or exchanging the medium in which the L23a-reactive T cells are cultured with a medium containing the test substance. Preferably, a control group with no contact with the test substance is provided.

Also in the step of bringing the L23a-reactive T cells into contact with L23a or a fragment thereof, the L23a-reactive T cells may be brought into contact with L23a or a fragment thereof by any method and the method is not particularly limited. The L23a-reactive T cells may be brought into contact with L23a or a fragment thereof by, for example, adding L23a or a fragment thereof to the medium in which the L23a-reactive T cells are cultured, or exchanging the medium in which the L23a-reactive T cells are cultured with a medium containing L23a or a fragment thereof.

Either of the steps of bringing the test substance into contact with the L23a-reactive T cells and of bringing the L23a-reactive T cells into contact with L23a or a fragment thereof may be performed first, or alternatively these steps may be simultaneously performed. In other words, the L23a-reactive T cells may first be brought into contact with the test substance and then brought into contact with L23a or a fragment thereof; or alternatively, the L23a-reactive T cells may first be brought into contact with L23a or a fragment thereof and then brought into contact with the test substance; or alternatively, the L23a-reactive T cells may be brought into contact with the test substance and L23a or a fragment thereof simultaneously.

In the step of measuring the activation level of the L23a-reactive T cells, the measurement items are not particularly limited as long as the items serve as indicators of the activation level of the L23a-reactive T cells. Suitable measurement items may be, for example, the level of a proinflammatory cytokine secreted from the L23a-reactive T cells, the level of proliferation of the L23a-reactive T cells, the level of an activated surface marker of the L23a-reactive T cells, and/or the like.

In cases where the level of a proinflammatory cytokine secreted from the L23a-reactive T cells is measured as the indicator, the level of the proinflammatory cytokine is measured in the step of measuring the activation level of the L23a-reactive T cells. The proinflammatory cytokine to be measured is not particularly limited, and may be one or more proinflammatory cytokines appropriately selected from the above exemplified known proinflammatory cytokines. The method for measuring the proinflammatory cytokine is not particularly limited, and is appropriately selected from known methods for measuring the proinflammatory cytokine to be measured (for example, western blotting, EIA, ELISA, RIA, etc.).

Then, the test substance-dependent change in the secretion level of the proinflammatory cytokine is analyzed in the step of analyzing the test substance-dependent change in the activation level of the L23a-reactive T cells. In this step, the analysis method for the test substance-dependent change in the secretion level of the proinflammatory cytokine is not particularly limited. The analysis may be performed by, for example, comparing the secretion level of the proinflammatory cytokine with that of a control group with no contact with the test substance. When the comparison results show a reduction of the proinflammatory cytokine secretion in the group contacted with the test substance, the test substance is selected as a substance that inhibits the activation of autoimmune arthritogenic T cells. The degree of the reduction of the proinflammatory cytokine secretion by the test substance is not particularly limited, but preferably, for example, the test substance reduces the proinflammatory cytokine secretion to 50% or less of that of the cells with no contact with the test substance, and more preferably the test substance reduces the proinflammatory cytokine secretion to 25% or less of that of the cells with no contact with the test substance.

In cases where the level of proliferation of the L23a-reactive T cells is measured as the indicator, the level of the proliferation is measured in the step of measuring the activation level of the L23a-reactive T cells. The method for measuring the level of proliferation of the L23a-reactive T cells is not particularly limited, and is appropriately selected from known methods for measuring cell proliferation. Examples of the measurement method include tritiated thymidine incorporation, and cell division detection by CFSE labeling.

Then, the test substance-dependent change in the level of cell proliferation is analyzed in the step of analyzing the test substance-dependent change in the activation level of the L23a-reactive T cells. In this step, the analysis method for the test substance-dependent change in the level of the cell proliferation is not particularly limited. The analysis may be performed by, for example, comparing the level of the cell proliferation with that of a control group with no contact with the test substance. When the comparison results show a reduction of the level of the cell proliferation in the group contacted with the test substance, the test substance is selected as a substance that inhibits the activation of autoimmune arthritogenic T cells. The degree of the reduction of the cell proliferation by the test substance is not particularly limited, but preferably, for example, the test substance reduces the cell proliferation to 50% or less of that of the cells with no contact with the test substance, and more preferably the test substance reduces the cell proliferation to 25% or less of that of the cells with no contact with the test substance.

In cases where the level of an activated surface marker of the L23a-reactive T cells is measured as the indicator, the level of the activated surface marker is measured in the step of measuring the activation level of the L23a-reactive T cells. The activated surface marker to be measured is not particularly limited, and examples thereof include CD154 etc. The method for measuring the surface marker is not particularly limited, and is appropriately selected from known methods for measuring a cell surface marker (for example, a method using a flow cytometer etc.).

Then, the test substance-dependent change in the level of the activated surface marker is analyzed in the step of analyzing the test substance-dependent change in the activation level of the L23a-reactive T cells. In this step, the analysis method for the test substance-dependent change in the level of the activated surface marker is not particularly limited. The analysis may be performed by, for example, comparing the level of the activated surface marker with that of a control group with no contact with the test substance. When the comparison results show a reduction of the level of the activated surface marker in the group contacted with the test substance, the test substance is selected as a substance that inhibits the activation of autoimmune arthritogenic T cells. The degree of the reduction of the activated surface marker by the test substance is not particularly limited, but preferably, for example, the test substance reduces the activated surface marker to 50% or less of that of the cells with no contact with the test substance, and more preferably the test substance reduces the activated surface marker to 25% or less of that of the cells with no contact with the test substance.

### Medicament containing a substance selected by screening

A substance selected by the screening method of the present invention is useful as an active ingredient of a prophylactic or therapeutic agent for autoimmune arthritis. Accordingly, the present invention includes a prophylactic or therapeutic medicament for autoimmune arthritis comprising, as an active ingredient, a substance that inhibits the activation of L23a-reactive T cells.

In cases where a substance selected by the screening method of the present invention is used as an active ingredient of a prophylactic or therapeutic medicament for autoimmune arthritis, the substance can be formulated into a pharmaceutical preparation by a conventional method. Examples of a pharmaceutical preparation for oral administration include solid or liquid dosage forms, in particular, tablets (including sugar-coated tablets and film-coated tablets), pills, granules, powders, capsules (including soft capsules), syrups, emulsions, suspensions, etc. These pharmaceutical preparations are produced by a known method and may contain a carrier, a diluent, and/or an excipient that is usually used in the pharmaceutical field. Examples of the carrier or excipient for tablets include lactose, starch, sucrose, magnesium stearate, etc. Examples of a pharmaceutical preparation for parenteral administration include injections, suppositories, etc. Examples of the injections include intravenous injections, hypodermic injections, intradermal injections, intramuscular injections, intravenous infusions, and intraarticular injections. Such an injection is prepared in accordance with a known method, for example, by dissolving, suspending or emulsifying the above substance that inhibits the activation of L23a-reactive T cells or a salt thereof into an aseptic aqueous or oily liquid usually used for the preparation of an injection. Examples of the aqueous liquid for an injection include physiological saline, and an isotonic solution containing glucose and/or other auxiliary agents. The aqueous liquid may be used in combination with a suitable solubilizing agent such as an alcohol (e.g., ethanol etc.), a polyalcohol (e.g., propylene glycol, polyethylene glycol, etc.), and a nonionic surfactant (e.g., polysorbate 80, HCO-50, etc.). Examples of the oily liquid include a sesame oil and a soybean oil. The oily liquid may be used in combination with a solubilizing agent such as benzyl benzoate and benzyl alcohol. A suppository for rectal administration is prepared by mixing the above substance that inhibits the expression or function of SIK3 or a salt thereof with a usual base for a suppository.

The thus produced pharmaceutical preparations are safe and low toxic and can therefore be orally or parenterally administered to, for example, a human or a non-human mammal (e.g., mouse, rat, rabbit, sheep, pig, cattle, horse, bird, cat, dog, monkey, chimpanzee, etc.).

### Use of fragments

The present invention also provides a fragment of L23a. The fragment of the present invention can be used for the detection of an anti-L23a antibody and of L23a-reactive CD4⁺ T cells in the identification method of the present invention. The fragment of the present invention can also be used for basic research, including the analysis of CD4⁺ T cells that induce arthritis in an autoantibody-independent manner, and the mechanistic analysis of autoantibody-independent arthritis.

### EXAMPLES

The present invention will be described in more detail below with reference to Examples, but the present invention is not limited thereto.

### Cloning of T cell receptors (TCRs) of arthritis-inducing T cells

The inventors created eFOX mice with knock-in of a fusion gene encoding a fusion protein of EGFP and the regulatory T cell-specific transcription factor Foxp3. The regulatory T cells of eFOX mice are readily detectable by the EGFP signals. eFOX mice were crossed to SKG mice, the autoimmune arthritis mouse model, to generate eFOX SKG mice. The mice were maintained in the SPF environment. eFOX SKG mice spontaneously developed arthritis after about three months of breeding.

Effector CD4⁺ T cells were isolated from the joint sites of eFOX SKG mice that developed arthritis. Briefly, the synovial tissue was removed from the ankle joints and dissociated in RPMI 1640 containing Liberase™ (Roche) at 37°C for 1 hour. The tissue was passed through a nylon mesh to prepare a single cell suspension. GFP-CD4⁺ T cells were single-cell sorted into 96-well PCR plates with a Cyclone device on a Moflo cell sorter. The plates were stored at -80°C until use.

TCRs were cloned from the sorted single GFP-CD4⁺ T cells by nested PCR. Briefly, to the wells was added 5 µL/well of a reaction mix [100 mg/mL gelatin, 0.1% Triton X-100, 4 U/mL RNasin, 125 ng OligodT (Promega), 0.5 mM dNTPs, 5 U/mL Multiscribe RT enzyme, 1× RT Buffer (High Throughput cDNA kit, Applied Biosystems)], and reverse transcription was performed under the conditions of at 25°C for 10 minutes, 37°C for 2 hours and 85°C for 5 minutes. Two µL aliquots of the reaction mixture were each used for amplification of the TCR α and β chain genes. The first PCR reaction was performed as follows: 95°C for 1 minute; 5 cycles of 95°C for 30 seconds, 50°C for 1 minute and 72°C for 30 seconds; 30 cycles of 95°C for 30 seconds, 58°C for 1 minute and 72°C for 30 seconds; and 72°C for 5 minutes. The second PCR reaction was performed under the same conditions using inner primers. The cloned TCR genes were subcloned into a retroviral vector for TCR expression (Lennon, G. P. et al. T cell islet accumulation in type 1 diabetes is a tightly regulated, cell-autonomous event. Immunity 31, 643-653, doi: 10.1016/j.immuni.2009.07.008 (2009)). The restriction enzyme recognition sites used for the subcloning of the α and β chains were MfeI-StuI and BamHI-XhoI, respectively. Thus, the TCR genes were cloned from each of about 150 single cells.

The above constructed retroviral vectors were used to construct retrogenic mice. The vector plasmids were separately transfected into the Plat-E cell line using Fugene 6 transfection reagent (Roche). Retroviral supernatants were harvested 48 and 72 hours after transfection and 0.45 µm filtered to remove cell debris.

RAG2 knockout mice with deletion of T and B cells and with SKG mutation (generated by crossing SKG mice and RAG2 knockout mice) were injected intraperitoneally with 5-fluorouracil at 150 mg/kg. After 4 days, the bone marrow cells were harvested and subjected to removal of the red blood cells. The bone marrow cells were then cultured at 2 × 10⁶ cells/mL in a DMEM solution supplemented with 20% fetal calf serum, penicillin, streptomycin and 2-ME in the presence of IL-3 (20 ng/mL), IL-6 (40 ng/mL) and SCF (50 ng/mL) (Peprotech). On days 2 and 3 of culture, the bone marrow cells were retrovirally infected by exchanging the medium with a medium containing each of the retroviral supernatants supplemented with IL-3 (20 ng/mL), IL-6 (40 ng/mL), SCF (50 ng/mL) and polybrene (4 µg/mL) and spinning the cells at 700 g at 32°C for 90 minutes. On day 4 of culture, recipient RAG2 knockout mice were irradiated with 600 rad and intravenously injected with the bone marrow cells. About 30 types of retrogenic mice each infected with one type of retrovirus were constructed.

The constructed retrogenic mice each exclusively express, as the TCR on T cells, a single TCR pair that was introduced and expressed by the retroviral vector. The arthritogenic capacity of the TCR of each retrogenic mouse can be determined by examining whether the retrogenic mouse develops arthritis.

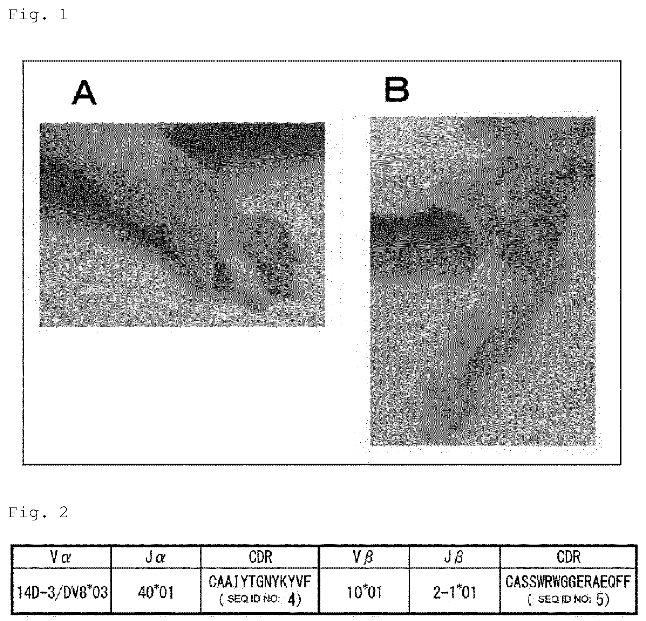

The constructed retrogenic mice were maintained in the SPF environment, and an individual that spontaneously developed arthritis was identified. Figs. 1A and 1B are the photographs of the mouse that developed arthritis. The retrovirus that was used to infect the mouse was analyzed to determine the amino acid sequence of the arthritogenic TCR (see Fig. 2). In Fig. 2, the sequences indicated by "14D-3/DV8*03", "40*01", "10*01" and "2-1*01" are obtainable from, for example, the database IMGT/LIGM-DB (http://www.imgt.org/ligmdb/).

### Identification of antigens recognized by arthritogenic TCR

The candidate antigens recognized by the arthritogenic TCR identified above were narrowed as follows.

The above TCR gene identified to be causative of arthritis was transfected into the TCR expression-deficient T cell line, 5KC-73.8.20, with use of the above retroviral supernatant in the same manner as above. The T cell line is known to produce IL-2 in response to the stimulation from the TCR. Separately, antigen-presenting cells (APCs) were prepared from BALB/c mouse spleen cells using MACS beads (Miltenyi) by concentrating and recovering CD11c⁺ cells and CD11b⁺ cells with the MACS system.

Next, the synovial cells from the joints of SKG mice that developed arthritis were disrupted to prepare a lysate. The disrupted cell lysate was separated into bands by Native-PAGE. The bands were cut out and subjected to extraction of the proteins contained in the bands.

The above T cell line carrying the introduced TCR gene (5 × 10⁴ cells/well) was seeded with the APCs (5 × 10⁴ cells/well) in 96-well round bottom plates, and the cells were co-cultured with the addition of each of the proteins extracted from the bands. After 24-hour culture, IL-2 concentrations in the supernatants were measured by ELISA (eBioscience). The bands with significant elevation of the IL-2 concentrations were selected as the candidate bands.

The proteins extracted from the candidate bands were subjected to mass spectrometry. From the MS results, the candidate proteins were narrowed down to several.

From each of the several candidate proteins, a recombinant protein was produced and then added to the co-culture system of the T cell line and the APCs to determine whether the proteins were the antigens recognized by the arthritogenic TCR using an increase of IL-2 concentration as an indicator. The results revealed that the addition of L23a (60S ribosomal protein L23a (RPL23A)) increases the IL-2 concentration.

### Activation of arthritogenic TCR by L23a

The coding region of the DNA encoding human L23a (SEQ ID NO: 2, ACCESSION: NM_000984) was inserted into the expression vector pGEX-6P1. The expression vector was transfected into *Escherichia coli,* and the bacteria were cultured in LB medium at 37°C. After growth, the bacteria were cultured in the presence of 0.1 mM IPTG at 25°C for 5 hours for induction of protein expression. The bacteria were then lysed in PBS containing 1% Triton X-100, and the resulting solution was sonicated to dissolve the proteins. The resulting protein solution was subjected to purification on Glutathione-Sepharose fastflow beads to give recombinant GST-L23a. As a control, a recombinant GST protein was produced in the same manner as above using pGEX-6P1 vector without any inserted gene.

The test groups as shown in Table 1 were prepared using the T cell line carrying the introduced TCR gene, the antigen-presenting cells (APCs), the recombinant GST-L23a, the recombinant GST, and a lysate of a disrupted mouse myeloma cell (B cell) line P3U1 (positive control). The T cell line and the APCs were seeded on 96-well round bottom plates, and a medium containing each of the test substances was added. After 24-hour culture, the IL-2 concentrations in the supernatants were measured by ELISA (eBioscience). The disrupted P3U1 cell lysate was selected as a positive control because it had been previously studied by the inventors and revealed that it reacts with the arthritogenic TCR cloned herein. The disrupted cell lysate was prepared as follows. Briefly, 1 × 10⁷ P3U1 cells were suspended in 1 mL of PBS, frozen with liquid nitrogen and thawed. Thirty seconds of sonication was repeated 3 times and centrifugation was performed at 15, 000 rpm for 15 minutes. The supernatant was used as a disrupted cell lysate.

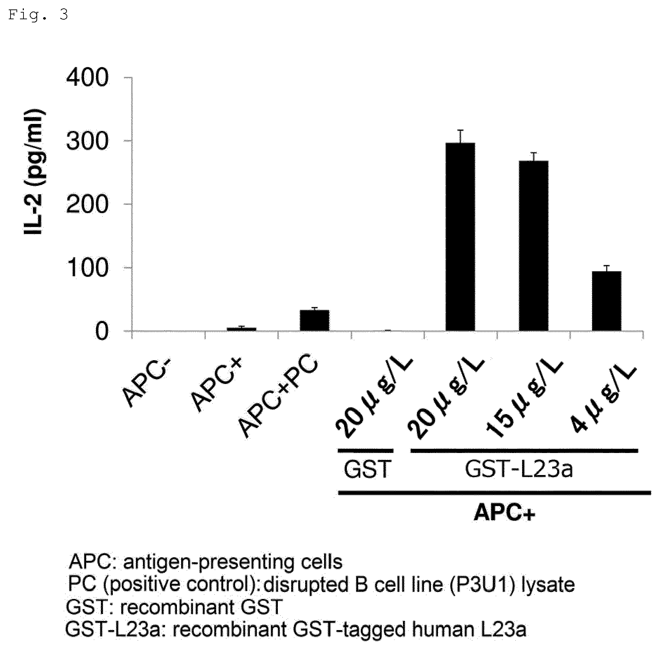

The results of the study are shown in Fig. 3. As apparent from Fig. 3, GST-L23a activates the arthritogenic TCR in a concentration-dependent manner.

**Table 2**

| Test group | T cell line 5 × 10⁴ cells/well | APCs 5 × 10⁴ cells/well | Disrupted P3U1 cell lysate | Recombinant GST | Recombinant GST-RPL23A |
|---|---|---|---|---|---|
| APC- | ✔ | - | - | - | - |
| APC+ | ✔ | ✔ | - | - | - |
| APC+/PC | ✔ | ✔ | 200 µg/mL | - | - |
| APC+/GST | ✔ | ✔ | - | 20 µg/mL | - |
| APC+/GST-RPL23A | ✔ | ✔ | - | - | 20 µg/mL |
| APC+/GST-RPL23A | ✔ | ✔ | - | - | 15 µg/mL |
| APC+/GST-RPL23A | ✔ | ✔ | - | - | 4 µg/mL |

### Identification of antigen fragments of L23a

Various peptides of about 20 amino acid residues were synthesized based on the amino acid sequence of L23a (SEQ ID NO: 1) by the conventional method. Experiments were conducted in the same manner as above using the T cell line carrying the introduced TCR gene, the antigen-presenting cells (APCs), the various synthesized peptides and the recombinant GST-L23a (full-length L23a). Briefly, the T cell line and the APCs were seeded on 96-well round bottom plates, and a medium containing each of the synthesized peptides and GST-L23a was added. After 24-hour culture, the IL-2 concentrations in the supernatants were measured by ELISA (eBioscience). The concentration of the peptides was 50 µg/mL and the concentration of GST-L23a was 100 µg/mL.

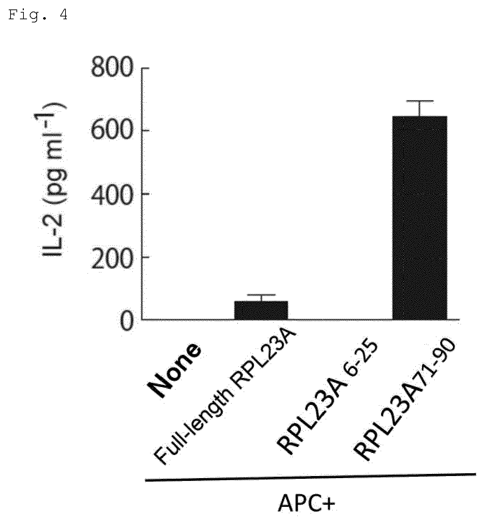

The results of the study are shown in Fig. 4. As apparent from Fig. 4, L23a71-90 significantly increased the IL-2 concentration. On the other hand, L23a6-25 did not increase the IL-2 concentration. Although not shown in the figure, the peptides other than L23a71-90 showed the same results as that of L23a6-25. The overall results revealed that the fragment consisting of the amino acids at positions 71 to 90 of L23a (LDHYAIIKFPLTTESAMKKI, SEQ ID NO: 3) is the antigen recognized by the arthritogenic TCR.

### Detection of anti-L23a antibody in the serum of retrogenic mice expressing arthritogenic TCR

The blood was collected from the retrogenic mice that developed arthritis (the retrogenic mice expressing arthritogenic TCR that recognizes L23a), and the serum was separated.

The disrupted P3U1 cell lysate, the recombinant GST-L23a and a recombinant GST-histone 1.2 were separately added to 2ME-containing sample buffer, and the mixtures were heated at 95°C for 5 minutes. The mixtures were electrophoresed on a 15% polyacrylamide gel at 20 mA for 75 minutes. The separated proteins were transferred to Immobilon-P Transfer Membrane (Millipore) at 90 V for 30 minutes. After the transfer, the membrane was reacted with the serum at a 200-fold dilution. After washing, the membrane was reacted with a HRP-conjugated anti-mouse IgG1 antibody at a 2, 000-fold dilution as a secondary antibody. After washing, the color was developed with ECL Prime Western Blotting Detection Reagent and detected with LAS-4000mini.

Histone H1.2 was included in the candidate antigens recognized by the arthritogenic TCR cloned herein, but the protein was identified to have no reactivity to the arthritogenic TCR. Histone H1.2 was prepared as a recombinant protein tagged with GST in the same manner as the preparation of GST-L23a, by inserting the coding region of the DNA encoding mouse histone H1.2 (ACCESSION: NM_015786) into the expression vector pGEX-6P1.

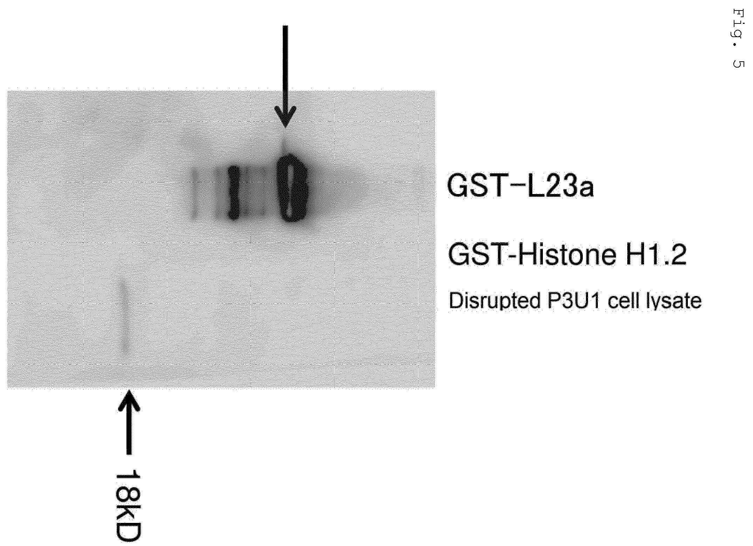

The results are shown in Fig. 5. In Fig. 5, the arrow at the left-hand side indicates the band of GST-L23a, and the arrow at the right-hand side indicates the band of the disrupted P3U1 cell lysate protein that was bound by the serum antibody. No detection of the antibody binding to GST-histone 1.2 indicated the existence of a large amount of the L23a-recognizing antibody in the serum of the retrogenic mice expressing the arthritogenic TCR that recognizes L23a.

### Detection of anti-L23a antibody in the sera of rheumatoid arthritis patients

The blood was collected from 374 rheumatoid arthritis patients and 80 healthy individuals without rheumatoid arthritis, and the sera were separated.

The recombinant GST-L23a and the recombinant GST were separately dissolved in PBS to prepare 200 ng/mL solutions. The solutions were separately added to the wells of PolySorp™ ELISA plates (Nunc) and allowed to be adsorbed at 4°C for 16 hours. After washing 5 times with PBS containing 0.05% Tween 20, the plates were blocked with PBS containing 1% BSA for 1 hour. After washing 5 times in the same manner as above, the sera diluted to 500-fold in PBS containing 1% BSA were separately added to each well in a volume of 100 µL. The plates were left to stand at room temperature for 2 hours. After washing 5 times, a HRP-conjugated anti-human IgG (H+L) (diluted to 4, 000-fold in PBS containing 1% BSA) was added to each well and the plates were left to stand at room temperature for 1 hour. After washing 5 times, TMB was added to each well, and 15 minutes after the addition, the OD450nm was measured. The OD values of the anti-L23a antibody were calculated by subtracting the OD values of GST from the OD values of GST-L23a.

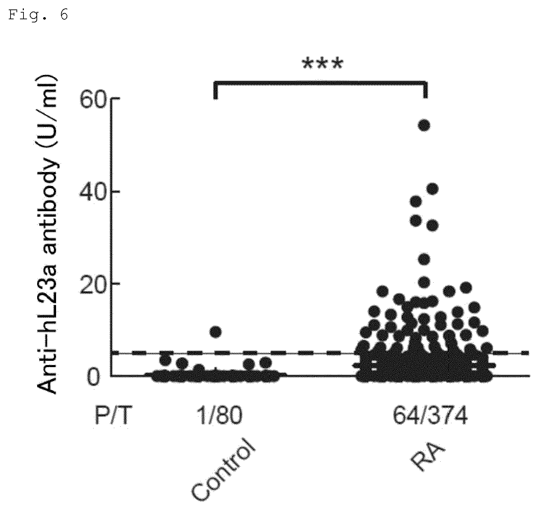

The results are shown in Fig. 6. In the figure, Control indicates the healthy individuals, and RA indicates the rheumatoid arthritis patients. The unit (U/mL) was defined as a quantity relative to arbitrarily selected one positive serum. The cutoff for normal values was defined as less than or equal to +3SD from the mean value of the healthy individuals (on or below the broken line in the chart). The values exceeding the cutoff was determined to be positive. As shown in Fig. 6, only one out of the 80 healthy individuals was positive, whereas 64 out of the 374 rheumatoid arthritis patients were positive. Significant difference was observed by t-test (p value = 0.0012).

### Detection of L23a-reactive T cells in the synovial fluid of rheumatoid arthritis patient

Synovial fluid was collected from one rheumatoid arthritis patient. Blood was collected from one healthy individual. Mononuclear cells were separated from the synovial fluid and the blood, respectively. Briefly, the synovial fluid and the blood were separately centrifuged with the addition of Ficoll at 2, 200 rpm for 20 minutes to separate and recover mononuclear cells. The mononuclear cells were seeded on 96-well plates at 1 × 10⁶ cells/well, and the recombinant GST or the recombinant GST-L23a was added to the medium at 100 µg/mL. The cells were cultured for 24 hours. Six hours before the end of culture, Brefeldin A was added so as to be 1 µg/mL to stop the secretion of cytokines. After harvesting of the cells, IL-17A and IFN-γ were stained by common cytokine staining and detected with a flow cytometer.

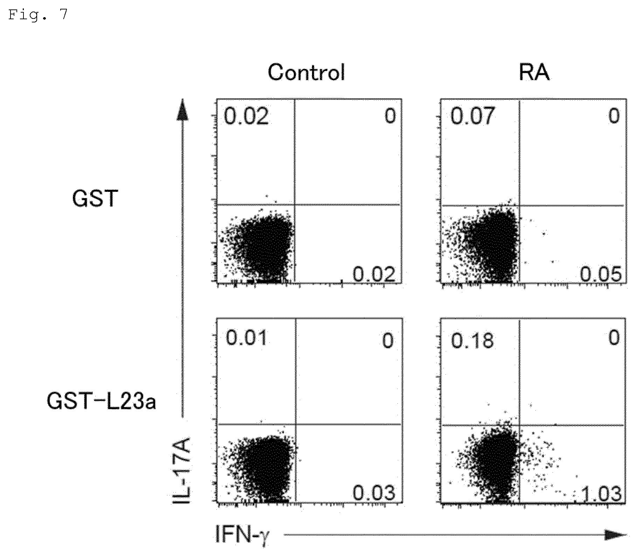

The results are shown in Fig. 7. In the synovial fluid of the rheumatoid arthritis patient, the cells secreting the proinflammatory cytokines IL-17A and IFN-γ in response to GST-L23a were detected (bottom right). On the other hand, in the blood of the healthy individual, no cells secreting the proinflammatory cytokines in response to GST-L23a was detected.

### Detection of anti-L23a antibody in the sera of anti-CCP antibody-negative rheumatoid arthritis patients

More than 90% of rheumatoid arthritis patients are positive for anti-CCP (cyclic citrullinated peptide) antibody, and accordingly anti-CCP antibody is widely used as a very sensitive diagnostic marker for rheumatoid arthritis. However, it has been known that about 10% of rheumatoid arthritis patients are negative for the antibody.

Based on these knowledge, the sera were separated from the blood collected from 59 rheumatoid arthritis patients who were anti-CCP antibody negative, and the sera were subjected to measurement of serum anti-L23a antibody by the same method (ELISA) as in the above "Detection of anti-L23a antibody in the sera of rheumatoid arthritis patients".

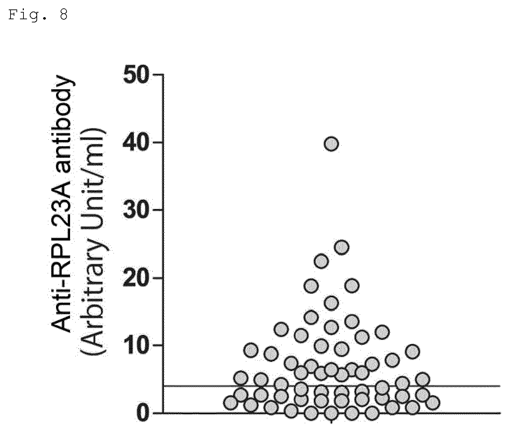

The results are shown in Fig. 8. The cutoff for normal values was defined in the same manner as in the above "Detection of anti-L23a antibody in the sera of rheumatoid arthritis patients". As shown in Fig. 8, 32 out of the 59 patients (54%) were positive. Since anti-L23a antibody was detected in more than half of the anti-CCP antibody-negative rheumatoid arthritis patients, this study confirmed the usefulness of the identification method of the present invention.

The present invention is not limited to each of the embodiments and Examples described above, and various modifications are possible within the scope of the claims. Embodiments obtainable by appropriately combining the technical means disclosed in the different embodiments are also included in the technical scope of the present invention. The entire contents of the scientific literature and the patent literature cited herein are incorporated herein by reference.

## Claims

1. A method for identifying the presence or likelihood of autoimmune arthritis, the method comprising detecting
(1) an anti-ribosomal protein L23a antibody or
(2) ribosomal protein L23a-reactive CD4⁺ T cells in a sample derived from a subject.

2. The identification method according to claim 1, wherein the ribosomal protein L23a is a protein consisting of an amino acid sequence of SEQ ID NO: 1.

3. The identification method according to claim 1 or 2, wherein the subject is anti-CCP antibody negative.

4. Use of ribosomal protein L23a or a fragment thereof for the identification of the presence or likelihood of autoimmune arthritis, the use comprising detecting an anti-ribosomal protein L23a antibody or ribosomal protein L23a-reactive CD4⁺ T cells by using ribosomal protein L23a or a fragment thereof.

5. The use according to claim 4, wherein the ribosomal protein L23a is a protein consisting of an amino acid sequence of SEQ ID NO: 1.

6. The use according to claim 4, wherein the ribosomal protein L23a fragment comprises the amino acids at positions 71 to 90 of SEQ ID NO: 1.

7. A method for screening for a substance that inhibits the activation of autoimmune arthritogenic T cells, the method comprising the steps of
bringing a test substance into contact with ribosomal protein L23a-reactive T cells,
bringing the T cells into contact with ribosomal protein L23a or a fragment thereof,
measuring the activation level of the T cells, and analyzing the test substance-dependent change in the activation level of the T cells.

8. The screening method according to claim 7, wherein the step of measuring the activation level of the T cells comprises measuring the level of a proinflammatory cytokine secreted from the T cells, the level of proliferation of the T cells or the level of an activated surface marker of the T cells.

9. The screening method according to claim 7 or 8, wherein the ribosomal protein L23a is a protein consisting of an amino acid sequence of SEQ ID NO: 1.

10. The screening method according to claim 7 or 8, wherein the ribosomal protein L23a fragment comprises the amino acids at positions 71 to 90 of SEQ ID NO: 1.

11. A ribosomal protein L23a fragment consisting of part of an amino acid sequence of SEQ ID NO: 1 and comprising at least 5 contiguous amino acid residues of the amino acid sequence of SEQ ID NO: 1.

12. The fragment according to claim 11, comprising the amino acids at positions 71 to 90 of SEQ ID NO: 1.


SEQUENCE LISTING

<110>  Kyoto University

<120>  Methods to determine existence of arthritogenic T-cells and methods
of screening for substances inhibiting activation of arthritogenic T cells

<130>  K29F5019

<150>  JP 2013-159815
<151>  2013-07-31

<160>  5

<170>  PatentIn version 3.1

<210>  1
<211>  156
<212>  PRT
<213>  Homo sapiens

<400>  1

<210> 2
<211> 979
<212> DNA
<213> Homo sapiens

<400> 2
   agcattggag acccttttca caagatggcg ccgaaagcga agaaggaagc tcctgcccct 60
   cctaaagctg aagccaaagc gaaggcttta aaggccaaga aggcagtgtt gaaaggtgtc 120
   cacagccaca aaaagaagaa gatccgcacg tcacccacct tccggcggcc gaagacactg 180
   cgactccgga gacagcccaa atatcctcgg aagagcgctc ccaggagaaa caagcttgac 240
   cactatgcta tcatcaagtt tccgctgacc actgagtctg ccatgaagaa gatagaagac 300
   aacaacacac ttgtgttcat tgtggatgtt aaagccaaca agcaccagat taaacaggct 360
   gtgaagaagc tgtatgacat tgatgtggcc aaggtcaaca ccctgattcg gcctgatgga 420
   gagaagaagg catatgttcg actggctcct gattacgatg ctttggatgt tgccaacaaa 480
   attgggatca tctaaactga gtccagctgc ctaattctga atatatatat atatatatct 540
   tttcaccata tacatgcctg tctgtcaatt tctggttggg ctgggaggcc acacacacac 600
   actgacatga cagggcttgg gcaagactcc tgttctactt atccttttga aatacctcac 660
   cctgccactc caccatgtat gatcattcca gagatctttg tgactagagt tagtgtccta 720
   ggaaaaccag aactcagaac ttgcctccat ggttgagtaa caagctgtac aagaacccct 780
   tttatccctg gaagaggctg tgtatgaaac caatgcccag ggtttgaagg gtgttagcat 840
   ccatttcagg ggagtgtgga ttggctggct ctctggtagc attttgtcct cacacaccca 900
   tctactatgt ccaaccggtc tgtctgcttc cctcacccct tgcccaataa aggacaagga 960
   cttcagagga aaaaaaaaa 979


<210> 3
<211> 20
<212> PRT
<213> Artificial sequence

<220>
<223> antigen fragment

<400> 3

Leu Asp His Tyr Ala Ile Ile Lys Phe Pro Leu Thr Thr Glu Ser Ala
1               5                   10                  15


Met Lys Lys Ile
            20


<210> 4
<211> 13
<212> PRT
<213> Mus musculus

<400>  4

Cys Ala Ala Ile Tyr Thr Gly Asn Tyr Lys Tyr Val Phe
1               5                   10

<210>  5
<211>  16
<212>  PRT
<213>  Mus musculus

<400>  5

Cys Ala Ser Ser Trp Arg Trp Gly Gly Glu Arg Ala Glu Gln Phe Phe
1               5                   10                  15